# EUROPÄISCHE PATENTANMELDUNG

(11) **EP 2 382 967 A1**
(43) Veröffentlichungstag der Anmeldung: **02.11.2011**
(21) Anmeldenummer: 10004251.4
(22) Anmeldetag: 21.04.2010
(51) Int. Cl.: A61K 9/14, A61K 9/20, A61K 31/165

(54) **Aliskiren in Form einer festen Dispersion**

(71) Anmelder: ratiopharm GmbH, 89079 Ulm (DE)
(72) Erfinder: Stephan, Ralph, 88370 Ebenweiler (DE); Meergans, Dominique, 81477 München (DE)
(74) Vertreter: TER MEER - STEINMEISTER & PARTNER GbR

(57) **Zusammenfassung**

Die Erfindung betrifft ein Intermediat, enthaltend Aliskirenbase und Trägerstoff, wobei das Aliskiren in Form einer festen Dispersion vorliegt. Die Erfindung betrifft ferner Verfahren zur Herstellung einer festen Dispersion von Aliskiren und pharmazeutische Formulierungen enthaltend Aliskiren in Form einer festen Dispersion.

## Beschreibung

Die Erfindung betrifft ein Intermediat, enthaltend Aliskiren als freie Base und Trägerstoff, wobei das Aliskiren in Form einer festen Dispersion vorliegt. Die Erfindung betrifft ferner Verfahren zur Herstellung einer festen Dispersion von Aliskirenbase und pharmazeutische Formulierungen, insbesondere Tabletten, enthaltend Aliskirenbase in Form einer festen Dispersion.

Aliskiren (lUPAC-Name: (2S,4S,5S,7S)-5-amino-N-(2-carbamoyl-2-methyl-propyl)-4-hydroxy-7-{[4-methoxy-3-(3-methorypropoxy)phenyl]methyl}-8-methyl-2-propan-2-yl-nonanamid) ist ein direkter Renin-Inhibitor, der zur Senkung von erhöhtem Blutdruck eingesetzt wird. Aliskiren hat die folgende chemische Struktur:

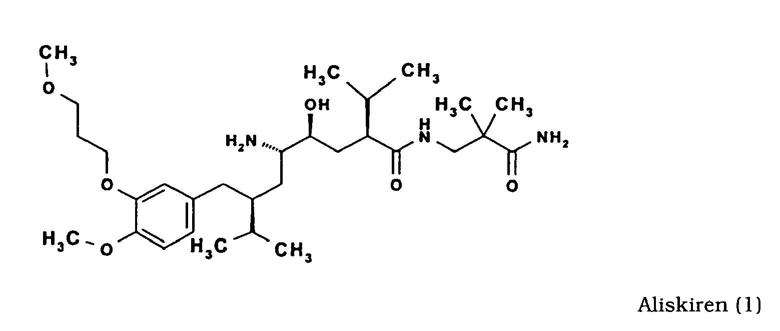

Eine Synthese von Aliskiren in Form des Hemifumaratsalzes ist in EP 0 678 503 A1 beschrieben. Es hat sich jedoch herausgestellt, dass Aliskiren, insbesondere in Form des Hemifumaratsalzes, schwierig zu formulieren ist, da kristallines Aliskiren unter anderem hygroskopisch ist und eine schlechte Fließfähigkeit aufweist, siehe WO 2005 /089729. Deshalb wurde in WO 2009/64479 auch amorphes Aliskiren vorgeschlagen. Problematisch ist hierbei jedoch, dass die Stabilität, verglichen mit dem kristallinen Zustand, nachteilig ist (siehe WO 2005/089729, S. 2).

Aufgabe der vorliegenden Erfindung war es daher, die vorstehend genannten Nachteile zu überwinden. Es soll der Wirkstoff in einer Form bereitgestellt werden, die eine gute Fließfähigkeit aufweist und somit eine gute Verpressung zu Tabletten gewährleistet. Es soll zudem der Wirkstoff in einer Form bereitgestellt werden, die nicht zur Agglomeration neigt. Ferner soll eine gleichmäßige Verteilung des Wirkstoffs gewährleistet sein. Eine Mikronisierung des Wirkstoffs soll vermieden werden, um die mit der Mikronisierung üblicherweise einhergehenden Nachteile zu vermeiden.

Ferner soll Aliskiren in einer Form bereitgestellt werden, die eine hohe Gleichförmigkeit des Gehalts (*Content Uniformity*) ermöglicht, insbesondere bei hohem Wirkstoffanteil (*drug load*).

Die Erfinder der vorliegenden Anmeldung waren bei der Entwicklung von Aliskiren-Formulierungen weiterhin mit der Tatsache konfrontiert, dass kristallines Aliskirenhemifumarat - wie in WO 2009/064479 beschrieben - in zehn verschiedenen polymorphen Formen existieren kann. Die unterschiedlichen Polymorphe sind jedoch zum Teil nicht stabil, sondern neigen dazu, sich in andere polymorphe Formen umzuwandeln, z.B. bei Wärmeinwirkung oder in feuchter Umgebung. Zudem weisen die unterschiedlichen polymorphen Formen unterschiedliche Löslichkeitsprofile auf.

Das unterschiedliche Löslichkeitsprofil führt beim Patienten zu einem unerwünscht ungleichmäßigen Anfluten des Wirkstoffs. Aufgabe der vorliegenden Erfindung war es daher, Aliskiren in einer Form bereit zu stellen, die eine möglichst gleichmäßige Anflutung beim Patienten ermöglicht. Sowohl interindividuelle als auch intraindividuelle Abweichungen sollen weitgehend vermieden werden.

Weiterhin soll der Wirkstoff in einer Form bereitgestellt werden, die gute Löslichkeit und gute Bioverfügbarkeit bei zeitgleich guter Lagerstabilität gewährleistet.

Die Aufgaben konnten unerwartet durch Bereitstellen von Aliskiren in Form einer festen Dispersion gelöst werden, wobei Aliskiren nicht als pharmazeutisch verträgliches Salz, sondern in Form der freien Base verwendet wird.

Gegenstand der Erfindung ist daher ein Intermediat, enthaltend Aliskiren und Trägerstoff, wobei das Aliskiren in Form einer festen Dispersion vorliegt und bevorzugt nicht als pharmazeutisch verträgliches Salz, sondern als freie Base eingesetzt wird. Dieses erfindungsgemäße Intermediat stellt eine feste Dispersion von Aliskiren, insbesondere Aliskirenbase, in stabilisierter Form dar.

Gegenstand der Erfindung sind ferner verschiedene Verfahren zur Herstellung einer festen Dispersion von Aliskirenbase in Form des erfindungsgemäßen Intermediats.

Schließlich sind Gegenstand der Erfindung pharmazeutische Formulierungen, enthaltend die erfindungsgemäße Aliskirenbase in Form einer festen Dispersion beziehungsweise in Form des erfindungsgemäßen Intermediats.

Der Begriff "feste Dispersion" ist im Rahmen dieser Erfindung so zu verstehen, dass Aliskirenbase auf und/oder in einem Trägerstoff, der bevorzugt bei 25 °C in festem Aggregatzustand vorliegt, dispers verteilt ist.

In einer bevorzugten Ausführungsform ist Aliskirenbase auf und/oder in dem Trägerstoff im Wesentlichen homogen verteilt. Dies bedeutet üblicherweise, dass Intermediate dieser bevorzugten Ausführungsform eine Gleichförmigkeit des Gehalts (*Content Uniformity*) mit einer Standardabweichung von ≤ 6% (d.h. 94 % bis 106 %) aufweisen. Besonders bevorzugt ist eine Ausführungsform, die eine Gleichförmigkeit des Gehalts mit einer Standardabweichung von ≤ 3% (d.h. 97 % bis 103 %) auszeichnet. Die "Content Uniformity" wird gemäß Ph.Eur.6.0, Abschnitt 2.9.6. bestimmt.

Das erfindungsgemäße Intermediat stellt somit eine feste Dispersion von Aliskirenbase in stabilisierter Form dar.

Im erfindungsgemäßen Intermediat ist Aliskirenbase auf den Trägerstoff aufgebracht und/oder eingelagert. Der Ausdruck "aufgebracht" bedeutet hierbei an der Oberfläche des Trägerstoffs durch physikochemische Wechselwirkungen, wie z. B. Van-der-Waals-, Wasserstoffbrücken-, oder Charge-Transfer-Wechselwirkungen, gebunden. Der Ausdruck "eingelagert" bedeutet hierbei im Inneren der Trägerstoffstruktur durch physikochemische Wechselwirkungen, wie z. B. Van-der-Waals-, Wasserstoffbrücken-, oder Charge-Transfer-Wechselwirkungen (zumindest teilweise) eingeschlossen. Es ist bevorzugt, dass mindestens 50%, mehr bevorzugt mindestens 70%, noch mehr bevorzugt mindestens 90%, insbesondere mindestens 95% des Trägerstoffs mit Aliskirenbase benetzt sind.

Die auf den Trägerstoff aufgebrachte und/oder in den Trägerstoff eingelagerte Aliskirenbase kann in flüssiger oder fester Form vorliegen. Bevorzugt liegt im erfindungsgemäßen Intermediat Aliskiren in fester Form vor.

Es ist ferner bevorzugt, dass das erfindungsgemäße Intermediat (enthaltend Aliskirenbase in Form einer festen Dispersion) im Wesentlichen kein kristallines Aliskiren enthält. Insbesondere enthält das erfindungsgemäße Intermediat weniger als 15 Gew.-%, mehr bevorzugt weniger als 5 Gew.-%, an kristalliner Aliskirenbase, bezogen auf das Gesamtgewicht der im Intermediat vorhandenen Aliskirenbase.

Insbesondere besteht das erfindungsgemäße Intermediat im Wesentlichen aus disperser Aliskirenbase und Trägerstoff. Sofern - wie nachstehend beschrieben - zusätzlich ein Kristallisationsinhibitor verwendet wird, so kann das erfindungsgemäße Intermediat im Wesentlichen aus molekulardisperser Aliskirenbase, Trägerstoff und Kristallisationsinhibitor bestehen. Der Ausdruck "im Wesentlichen" weist hier darauf hin, dass gegebenenfalls noch geringe Mengen Lösemittel etc. enthalten sein können. Bei dem Trägerstoff handelt es sich im Allgemeinen um einen Stoff, welcher geeignet ist, Aliskirenbase in Form einer festen Dispersion zu stabilisieren, insbesondere zu trägern und/oder einzuschließen. Bevorzugt handelt es sich bei dem Trägerstoff um ein Polymer oder um ein anorganisches Salz. Ferner umfasst der Trägerstoff auch Stoffe, die sich polymerähnlich verhalten.

In einer bevorzugten Ausführungsform handelt es sich bei dem Trägerstoff um einen nicht-wasserlöslichen Stoff. Bei dem nicht-wasserlöslichen Trägerstoff handelt es sich im Allgemeinen um einen im Europäischen Arzneibuch spezifizierten pharmazeutischen Hilfsstoff, welcher eine Wasserlöslichkeit von weniger als 50 mg/l, gemessen bei 25 °C, aufweist. Bevorzugt weist der nicht-wasserlösliche Stoff eine Löslichkeit von 20 mg/l oder weniger, mehr bevorzugt von 5 mg/ml oder weniger, insbesondere von 0,01 bis 4 mg/l, auf. Im Allgemeinen wird die Wasserlöslichkeit gemäß Säulenelutionsmethode nach EU-Richtlinie RL67-548-EWG, Anhang V, Kap. A6 bestimmt.

In einer weiteren bevorzugten Ausführungsform handelt es sich bei dem Trägerstoff um einen spröden Trägerstoff. Pharmazeutische Hilfsstoffe (z.B. Trägerstoffe) können generell über die Änderung der Partikelgestalt unter Pressdruck (Verpressung) klassifiziert werden: plastische Hilfsstoffe zeichnen sich durch plastische Deformation aus, während spröde Hilfsstoffe unter einwirkender Presskraft einen Bruch der Partikel in kleinere Partikel zeigen. Ein sprödes Verhalten des Trägerstoffs kann durch die Zunahme der Oberfläche in einem Pressling quantifiziert werden. Im Fachgebiet ist es üblich, die Sprödigkeit anhand des sogenannten "Yield Pressure" zu klassifizieren. Nach einer einfachen Klassifizierung sind dabei für plastische Substanzen die Werte für den "Yield Pressure" klein, bei brüchigen Substanzen dagegen groß [Duberg, M., Nyström, C., 1982, Studies on direct compression of tablets VI. Evaluation of methods for the estimation of particle fragmentation during compaction. Acta Pharm. Suec. 19, 421-436; Humbert-Droz P., Mordier D., Doelker E., Methode rapide de determination du comportement à la compression pour des etudes de preformulation. Pharm. Acta Helv., 57, 136-143 (1982)). Der "Yield pressure" beschreibt die Spannung, die erreicht werden muss, damit der Hilfsstoff (d.h. bevorzugt der Trägerstoff) anfängt plastisch zu fließen.

Bevorzugt wird der "Yield Pressure" über den reziproken Wert der Steigung des Heckel-plots ermittelt, wie in York, P., Drug Dev. Ind. Pharm. 18, 677 (1992) beschrieben. Die Messung erfolgt hierbei bevorzugt bei 25 °C und einer Deformationsgeschwindigkeit von 0,1 mm/s.

Im Rahmen der vorliegenden Erfindung gilt ein Hilfsstoff (insbesondere ein Trägerstoff) als nicht-spröder Hilfsstoff, wenn er einen "Yield Pressure" von maximal 120 MPa, bevorzugt maximal 100 MPa, besonders bevorzugt 5 bis 80 MPa, aufweist. Als spröder Hilfsstoff wird üblicherweise ein Hilfsstoff mit einem "Yield Pressure" von mehr als 80 MPa, bevorzugt mehr als 100 MPa, besonders bevorzugt mehr als 120 MPa, insbesondere mehr als 150 MPa, bezeichnet. Spröde Hilfsstoffe können einen "Yield Pressure" bis zu 300 MPa oder bis zu 400 MPa oder sogar bis zu 500 MPa aufweisen.

Beispiele für nicht-spröde Trägerstoffe sind Mannitol oder Stärke. Beispiele für spröde Trägerstoffe sind mikrokristalline Cellulose oder Dicalciumhydrogenphosphat. Spröde Trägerstoffe werden bevorzugt bei der Herstellung des erfindungsgemäßen Intermediats verwendet.

In einer weiteren bevorzugten Ausführungsform handelt es sich bei dem Trägerstoff um einen quellbaren Trägerstoff. Bei dem quellbaren Trägerstoff handelt es sich bevorzugt um ein quellbares Polymer oder um einen quellbaren Stoff mit polymerähnlichen Eigenschaften. Der quellbare Trägerstoff hat bevorzugt eine Quellungszahl von 1,2 bis 6,0, bevorzugt von 1,5 bis 4,5, mehr bevorzugt von 2,0 bis 4,0. Die Quellungszahl gibt das Volumen in Millilitern an, das 1 g Stoff einschließlich des gegebenenfalls anhaftenden Schleims nach Quellen in einer wässrigen Lösung nach 4 Stunden einnimmt. Die Quellungszahl wird nach Ph.Eur. 4. Ausgabe, Kapitel 2.8.4 bestimmt.

Insbesondere ist es bevorzugt, dass es sich bei dem Trägerstoff um einen spröden und nicht-wasserlöslichen oder um einen spröden, nicht-wasserlöslichen und quellbaren Trägerstoff handelt.

Des Weiteren kann es sich bei dem für die Herstellung des erfindungsgemäßen Intermediats verwendeten Trägerstoff um ein Polymer handeln, das bevorzugt eine Glasübergangstemperatur (Tg) von größer 20 °C. mehr bevorzugt von 25 °C. insbesondere von 35 °C, aufweist. Ein Polymer mit entsprechend gewählter Glasübergangstemperatur verhindert durch Immobilisierung die Rückbildung der Aliskirenbasedispersion zu größeren, und damit nicht mehr dispers verteilten Partikeln.

Als "Glasübergangstemperatur" (Tg) bezeichnet man die Temperatur, bei der amorphe oder teilkristalline Polymere vom festen Zustand in den flüssigen Zustand übergehen. Dabei tritt eine deutliche Änderung physikalischer Kenngrößen, z. B. der Härte und der Elastizität, ein. Unterhalb der Glasübergangstemperatur ist ein Polymer üblicherweise glasartig und hart, oberhalb der Glasübergangstemperatur geht es in einen gummiartigen bis zähflüssigen Zustand über. Die Bestimmung der Glasübergangstemperatur erfolgt im Rahmen dieser Erfindung mittels dynamischer Differenzkalorimetrie (DSC).

Hierzu kann ein Gerät von Mettler Toledo DSC 1 eingesetzt werden. Es wird mit einer Heizrate von 1-20 °C/min. bevorzugt 5-15 °C/min bzw. mit einer Kühlrate von 5-25 °C/min. bevorzugt 10 -20 °C/min gearbeitet.

Ferner weist das zur Herstellung des Intermediats verwendbare Polymer bevorzugt ein gewichtsmittleres Molekulargewicht von 1.000 bis 500.000 g/mol, mehr bevorzugt von 2.000 bis 90.000 g/mol, auf. Das gewichtsmittlere Molekulargewicht wird im Rahmen dieser Anmeldung bevorzugt mittels Gelpermeationschromatographie bestimmt. Wird das zur Herstellung des Intermediats verwendete Polymer in Wasser in einer Menge von 2 Gew.-% gelöst, so zeigt die resultierende Dispersion bevorzugt eine Viskosität von 0,1 bis 18 mPa•s, mehr bevorzugt von 0,5 bis 15 mPa•s, insbesondere von 1 bis 8 mPa•s, gemessen bei 25 °C und bevorzugt gemäß Ph. Eur. 6.0, Kapitel 2.2.10 bestimmt.

Bevorzugt werden zur Herstellung des Intermediats hydrophile Polymere verwendet. Darunter sind Polymere zu verstehen, die hydrophile Gruppen aufweisen. Beispiele für geeignete hydrophile Gruppen sind Hydroxy, Alkoxy, Acrylat, Methacrylat, Sulfonat, Carboxylat und quartäre Ammoniumgruppen. Hydroxygruppen sind bevorzugt.

Das erfindungsgemäße Intermediat kann beispielsweise folgende hydrophile Polymere als Trägerstoff umfassen: Polysaccharide, wie Hydroxypropylmethylcellulose (HPMC), Carboxymethylcellulose (CMC, insbesondere Natrium- und Calciumsalze), Ethylcellulose, Methylcellulose, Hydroxyethylcellulose, Ethylhydroxyethylcellulose, Hydroxypropylcellulose (HPC); mikrokristalline Cellulose, Polyvinylpyrrolidon, Polyvinylacetat (PVAC), Polyvinylalkohol (PVA), Polymere der Acrylsäure und deren Salze, Polyacrylamid, Polymethacrylate, Vinylpyrrolidon-Vinylacetat-Copolymere (beispielsweise Kollidon^{®} VA64, BASF), Polyalkylenglykole, wie Polypropylenglykol oder bevorzugt Polyethylenglykol, Co-blockpolymere des Polyethylenglykols, insbesondere Co-blockpolymere aus Polyethylenglykol und Polypropylenglykol (Pluronic^{®}, BASF), Polyethylenoxid sowie Gemische aus den genannten Polymeren.

Als Trägerstoff bevorzugt verwendet werden Polyvinylpyrrolidon, bevorzugt mit einem gewichtsmittleren Molekulargewicht von 10.000 bis 60.000 g/mol, insbesondere 12.000 bis 40.000 g/mol, Copolymer aus Vinylpyrrolidon und Vinylacetat, insbesondere mit einem gewichtsmittleren Molekulargewicht von 40.000 bis 70.000 g/mol und/oder Polyethylenglykol, insbesondere mit einem gewichtsmittleren Molekulargewicht von 2.000 bis 10.000 g/mol, sowie HPMC, insbesondere mit einem gewichtsmittleren Molekulargewicht von 20.000 bis 90.000 g/mol und/oder bevorzugt einem Anteil von Methylgruppen von 10 bis 35% und einem Anteil an Hydroxygruppen von 1 bis 35%. Ferner kann bevorzugt mikrokristalline Cellulose verwendet werden, insbesondere solche mit einer spezifischen Oberfläche von 0,7 - 1,4 m²/g. Die Bestimmung der spezifischen Oberfläche erfolgt mittels Gasadsorptions-methode nach Brunauer, Emmet und Teller.

Ferner umfasst der Trägerstoff auch feste, nicht-polymere Verbindungen, die bevorzugt polare Seitengruppen aufweisen. Beispiele hierfür sind Zuckeralkohole oder Disaccharide. Beispiele für geeignete Zuckeralkohole und/oder Disaccharide sind Mannitol, Sorbitol, Xylitol, Isomalt, Glucose, Fructose, Maltose und Gemische daraus. Der Begriff Zuckeralkohole umfasst hier auch Monosaccharide. In einer alternativen Ausführungsform können Cyclodextrine verwendet werden, z.B. alpha-Cyclodextrin, beta-Cyclodextrin oder gamma-Cyclodextrin.

Grundsätzlich sind auch Gemische aller genannten Trägerstoffe möglich.

Als besonders bevorzugter Trägerstoff wird mikrokristalline Cellulose verwendet.

Es ist vorteilhaft, wenn der Trägerstoff in partikulärer Form eingesetzt wird, wobei der mittlere Teilchendurchmesser (D50) der Trägerstoffteilchen 1 bis 250 µm, bevorzugt 10 bis 200 *µ*m, mehr bevorzugt 40 bis 150 *µ*m, beträgt. Der Ausdruck "mittlerer Teilchendurchmesser" bezieht sich im Rahmen dieser Erfindung stets auf den D50-Wert des volumenmittleren Teilchendurchmessers, der mittels Laserdiffraktometrie bestimmt wurde. Insbesondere wurde zur Bestimmung ein Mastersizer 2000 von Malvern Instruments verwendet (Nassmessung, mit Ultraschall 60 s, 2000 UpM), wobei die Auswertung nach dem Fraunhofer Modell erfolgt, und bevorzugt ein Dispergiermittel verwendet wird, in dem sich die zu messende Substanz bei 20 °C nicht löst. Der mittlere Teilchendurchmesser, der auch als D50-Wert der integralen Volumenverteilung bezeichnet wird, wird im Rahmen dieser Erfindung als der Teilchendurchmesser definiert, bei dem 50 Volumen-% der Teilchen einen kleineren Durchmesser haben als der Durchmesser, der dem D50-Wert entspricht. Ebenso haben dann 50 Volumen-% der Teilchen einen größeren Durchmesser als der D50-Wert.

In einer bevorzugten Ausführungsform enthält das erfindungsgemäße Intermediat Aliskirenbase und Trägerstoff, wobei das Gewichtsverhältnis von Aliskirenbase zu Trägerstoff 10 : 1 bis 1 : 10, mehr bevorzugt 5 : 1 bis 1 : 5, noch mehr bevorzugt 3 : 1 bis 1 : 3, insbesondere 2 : 1 bis 1 : 2, beträgt.

Es ist bevorzugt, dass Art und Menge des Trägerstoffs sowie Menge der Aliskirenbase so gewählt werden, dass das resultierende Intermediat eine Glasübergangstemperatur (Tg) von mehr als 20 °C aufweist, bevorzugt mehr als 30 °C. Die Glasübergangstemperatur des Intermediats sollte zudem unter 260 °C. bevorzugt unter 210 °C liegen.

In einer bevorzugten Ausführungsform enthalten die erfindungsgemäßen Intermediate zusätzlich zu Aliskirenbase und Trägerstoff noch einen Kristallisationsinhibitor auf der Basis eines anorganischen Salzes, einer organischen Säure oder eines hochmolekularen Polymers mit einem mittleren Molekulargewicht von größer 500.000 g/mol.

Diese als Kristallisationsinhibitor geeigneten Polymere werden im Rahmen dieser Erfindung auch als ,,hochviskoses Polymer" bezeichnet. Ihr gewichtsmittleres Molekulargewicht liegt üblicherweise unter 5.000.000 g/mol. Ein bevorzugtes hochviskoses Polymer ist Povidon.

Bevorzugt handelt es sich bei dem Kristallisationsinhibitor um Ammoniumchlorid, Zitronensäure, Silikate, wie bevorzugt Magnesiumaluminiummetasilikat (insbesondere vertrieben als Neusilin^{®}), oder Povidon K 90 (nach Ph.Eur. 6.0).

Der Kristallisationsinhibitor kann im Allgemeinen in einer Menge von 1 bis 30 Gew.-%, bevorzugt von 2 bis 25 Gew.-%, mehr bevorzugt von 5 bis 20 Gew.-%, bezogen auf das Gesamtgewicht des Intermediats, eingesetzt werden.

Die erfindungsgemäßen Intermediate sind durch verschiedene Herstellverfahren erhältlich. Je nach Herstellverfahren werden die Intermediate in unterschiedlichen Teilchengrößen erhalten. Üblicherweise liegen die erfindungsgemäßen Intermediate in partikulärer Form vor und weisen einen mittleren Teilchendurchmesser (D50) von 10 bis 350 µm, auf, abhängig vom jeweiligen Herstellungsverfahren.

Gegenstand der Erfindung ist ferner ein Verfahren zur Herstellung eines Intermediats, enthaltend Aliskiren und Trägerstoff, wobei Aliskirenbase als feste Dispersion vorliegt, umfassend die Schritte
(a) Lösen von Aliskirenbase in einem Lösungsmittel oder Lösungsmittelgemisch, sowie Inkontaktbringen der Lösung mit Trägerstoff,
(b) Entfernen des Lösungsmittels oder Lösungsmittelgemisches,
(c) gegebenenfalls Granulation des in Schritt (b) erhaltenen Gemisches.

Im Schritt (a) wird Aliskirenbase in einem Lösungsmittel oder Lösungsmittelgemisch gelöst, bevorzugt vollständig gelöst und mit dem Trägerstoff in Kontakt gebracht. Bevorzugt wird der vorstehend beschriebene Trägerstoff ebenfalls in diesem (d.h. Aliskirenbase-haltigem) Lösungsmittel oder Lösungsmittelgemisch suspendiert oder gelöst, bevorzugt suspendiert. Aliskirenbase und Trägerstoff werden somit bevorzugt in einem Lösungsmittel oder Lösungsmittelgemisch in Kontakt gebracht. Gegebenenfalls kann dies unter Rühren erfolgen, beispielsweise für 1 Minute bis 1 Stunde.

Als Lösungsmittel eignen sich z.B. Wasser, Alkohol (z.B. Methanol, Ethanol, Isopropanol), Dimethylsulfoxid (DMSO). Aceton, Butanol, Ethylacetat, Heptan, Pentanol oder Gemische daraus. Bevorzugt wird ein Gemisch aus Wasser und Alkohol, bevorzugt Ethanol, verwendet, wobei das Gewichtsverhältnis Alkohol : Wasser insbesondere 50 : 50 bis 99 : 1 beträgt.

In einer alternativen Ausführungsform kann Schritt (a) auch so ausgestaltet sein, dass die Aliskirenbaselösung auf den Trägerstoff aufgebracht wird, beispielsweise durch Aufsprühen.

Im anschließenden Schritt (b) erfolgt ein Entfernen des Lösungsmittels oder Lösungsmittelgemisches. Das Entfernen des Lösungsmittelgemisches kann beispielsweise durch erhöhte Temperatur (z.B. 50 bis 150 °C) und/oder durch reduzierten Druck (z.B. 0,001 bis 0,9 bar) erfolgen.

In einer bevorzugten Ausführungsform erfolgt im Schritt (b) eine Sprühtrocknung der Lösung, beziehungsweise bevorzugt Suspension, aus Schritt (a). Die Sprühtrocknung wird üblicherweise in einem Sprühturm durchgeführt. Beispielsweise ist ein Büchi B-191 geeignet (Büchi Labortechnik GmbH, Deutschland). Bevorzugt wird eine Eingangstemperatur von 50 °C bis 150 °C, mehr bevorzugt 70 °C bis 130 °C, gewählt. Die Luftmenge beträgt z.B. 500 bis 700 Liter/Stunde und der Aspirator läuft bevorzugt bei 80 bis 100%. In einer alternativen Ausführungsform erfolgt im Schritt (b) eine Gefriertrocknung der Lösung, beziehungsweise bevorzugt Suspension, aus Schritt (a).

Nach der Entfernung des Lösungsmittels erfolgt im optionalen Schritt (c) gegebenenfalls eine Granulation des in Schritt (b) erhaltenen Gemisches. Bevorzugt kann die Granulation auch während der Entfernung des Lösungsmittels erfolgen, d.h. die Schritte (b) und (c) erfolgen gleichzeitig.

Bevorzugt erfolgen die Schritte (b) und (c) in einem Wirbelschichtgranulator, beispielsweise in einem Glatt GPCG 3 (Glatt GmbH, Deutschland). Bevorzugt wird mit Zulufttemperaturen von 60 bis 80 °C, mit Produkttemperaturen von 30 bis 40 °C und mit einem Sprühdruck von 1 bis 1,5 bar gearbeitet.

Ebenfalls kann gemäß der vorstehenden alternativen Ausführungsform eine Aliskirenbaselösung auf in der Wirbelschicht befindliche Trägerstoffteilchen aufgebracht (z.B. aufgesprüht) werden, d.h. die Schritte (a), (b) und (c) können auch gleichzeitig erfolgen.

Das erfindungsgemäße Intermediat (d.h. die erfindungsgemäße Aliskirenbase in Form einer festen Dispersion) wird üblicherweise zur Herstellung einer pharmazeutischen Formulierung verwendet. Hierbei handelt es sich bevorzugt um eine pharmazeutische Formulierung zur oralen Verabreichung, insbesondere in Form einer Tablette.

Gegenstand der Erfindung ist daher eine pharmazeutische Formulierung, enthaltend erfindungsgemäßes Intermediat sowie pharmazeutische Hilfsstoffe.

Hierbei handelt es sich um die dem Fachmann bekannten Hilfsstoffe, beispielsweise solche, die im Europäischen Arzneibuch beschrieben sind.

Beispiele für verwendete Hilfsstoffe sind Sprengmittel, Trennmittel, Emulgatoren, Pseudo-Emulgatoren. Füllstoffe, Zusätze zur Verbesserung der Pulverfließfähigkeit, Gleitmittel, Netzmittel und/oder Schmiermittel. Gegebenenfalls können noch weitere Hilfsstoffe verwendet werden.

Das Verhältnis Wirkstoff zu Hilfsstoffe wird bevorzugt so gewählt, dass die resultierenden Formulierungen
10 bis 90 Gew.-%, mehr bevorzugt 20 bis 70 Gew.-%, insbesondere 35 bis 55 Gew.-% Aliskirenbase und
10 bis 90 Gew.-%, mehr bevorzugt 30 bis 80 Gew.-%, insbesondere 45 bis 65 Gew.-% pharmazeutisch verträgliche Hilfsstoffe enthalten.

Bei diesen Angaben wird die Menge an Trägerstoff, die gegebenenfalls zur Herstellung des erfindungsgemäßen Intermediats verwendet wurde, als Hilfsstoff gerechnet. Das heißt, die Menge an Wirkstoff bezieht sich auf die Menge an Aliskirenbase, die in der Formulierung enthalten ist.

Es hat sich gezeigt, dass die erfindungsgemäßen Intermediate dazu geeignet sind, sowohl als Basis für eine Darreichungsform mit sofortiger Freisetzung (*immediate release* oder kurz "IR") als auch mit modifizierter Freisetzung (*modified release* oder kurz "MR") dienen zu können.

In einer bevorzugten Ausführungsform für eine IR-Formulierung wird eine relativ hohe Menge an Sprengmittel verwendet. In dieser bevorzugten Ausführungsform enthält deshalb die erfindungsgemäße pharmazeutische Formulierung 5 bis 30 Gew.-%, mehr bevorzugt 10 bis 25 Gew.-%, insbesondere 12 bis 22 Gew.-% Sprengmittel, bezogen auf das Gesamtgewicht der Formulierung.

Als Sprengmittel werden im Allgemeinen Stoffe bezeichnet, die den Zerfall einer Darreichungsform, insbesondere einer Tablette, nach Einbringen in Wasser beschleunigen. Geeignete Sprengmittel sind z.B. organische Sprengmittel wie Carrageenan, Croscarmellose und Crospovidon. Ebenso verwendet werden alkalische Sprengmittel. Unter alkalischen Sprengmitteln sind Sprengmittel zu verstehen, die beim Lösen in Wasser einen pH-Wert von mehr als 7,0 erzeugen.

Mehr bevorzugt werden anorganische alkalische Sprengmittel verwendet, insbesondere Salze von Alkali- und Erdalkalimetallen. Bevorzugt sind hier Natrium, Kalium, Magnesium und Calcium zu nennen. Als Anionen sind z.B. Carbonat und/oder Hydrogencarbonat bevorzugt. Beispiele sind Natriumhydrogencarbonat, Natriumhydrogenphosphat, Calciumhydrogencarbonat und dergleichen.

Besonders bevorzugt wird Natriumhydrogencarbonat als Sprengmittel, insbesondere in den oben genannten Mengen, verwendet.

In einer bevorzugten Ausführungsform für eine MR-Formullerung wird eine relativ geringe Menge an Sprengmittel verwendet. In dieser bevorzugten Ausführungsform enthält deshalb die erfindungsgemäße pharmazeutische Formulierung 0 bis 10 Gew.-%, mehr bevorzugt 0,1 bis weniger als 5 Gew.-%, insbesondere 1 bis 4 Gew.-% Sprengmittel, bezogen auf das Gesamtgewicht der Formulierung.

Im Falle der MR-Formulierung ist Croscarmellose oder Crospovidon als Sprengmittel bevorzugt.

Ferner können für die MR-Formulierung die üblichen Retardierungstechniken verwendet werden.

Weiterhin enthält die pharmazeutische Formulierung (sowohl für IR als auch für MR) bevorzugt einen oder mehrere der vorstehend genannten Hilfsstoffe. Diese werden nachstehend näher erläutert.

In einer bevorzugten Ausführungsform enthält die erfindungsgemäße Formulierung 0 bis 8 Gew.-%, mehr bevorzugt 2 bis 7 Gew.-%, insbesondere 4 bis 6 Gew.-% Trennmittel, bezogen auf das Gesamtgewicht der Formulierung. Diese Ausführungsform wird insbesondere für die Herstellung von Tabletten verwendet.

Unter Trennmittel werden üblicherweise Stoffe verstanden, welche die Agglomeration im Kernbett vermindern. Beispiele sind Talkum, Silicagel, Polyethylenglykol (bevorzugt mit 2000 bis 10.000 g/mol gewichtsmittlerem Molekulargewicht) und/oder Glycerolmonostearat.

Die erfindungsgemäße Formulierung enthält bevorzugt Füllstoffe. Unter Füllstoffe sind im Allgemeinen Stoffe zu verstehen, die z.B. zur Bildung des Tablettenkörpers bei Tabletten mit geringen Wirkstoffmengen (z.B. kleiner 70 Gew.-%) dienen. Das heißt, Füllstoffe erzeugen durch "Strecken" der Wirkstoffe eine ausreichende Tablettiermasse. Füllstoffe dienen üblicherweise also dazu, eine geeignete Tablettengröße zu erhalten.

Beispiele für bevorzugte Füllstoffe sind Laktose, Laktosederivate, Stärke, Stärkederivate, behandelte Stärke, Talkum, Calciumphosphat. Dicalciumphosphat, Saccharose, Magnesiumcarbonat, Magnesiumoxid, Maltodextrin, Calciumsulfat, Dextrate, Dextrin, Dextrose, hydrogeniertes Pflanzenöl, Kaolin, Natriumchlorid, und/oder Kaliumchlorid. Ebenfalls kann mikrokristalline Cellulose und/oder silicierte mikrokristalline Cellulose (z.B. erhältlich als Prosolv^{®} Rettenmaier & Söhne, Deutschland) als Füllstoff verwendet werden.

Füllstoffe werden üblicherweise in einer Menge von 0 bis 60 Gew.-%, bevorzugt von 10 bis 40 Gew.-%, insbesondere von 15 bis 35 Gew.-%, bezogen auf das Gesamtgewicht der Formulierung, verwendet.

Ein Beispiel für einen Zusatz zur Verbesserung der Pulverfließfähigkeit ist disperses Siliciumdioxid, z.B. bekannt unter dem Handelsnamen Aerosil^{®}.

Zusätze zur Verbesserung der Pulverfließfähigkeit werden üblicherweise in einer Menge von 0,1 bis 3 Gew.-%, bezogen auf das Gesamtgewicht der Formulierung, verwendet.

Ferner können Schmiermittel verwendet werden. Schmiermittel dienen im Allgemeinen zur Verringerung der Gleitreibung. Insbesondere soll die Gleitreibung vermindert werden, die beim Tablettieren einerseits zwischen den sich in der Matrizenbohrung auf und ab bewegenden Stempeln und der Matrizenwand sowie andererseits zwischen Tablettensteg und Matrizenwand besteht. Geeignete Schmiermittel stellen z.B. Stearinsäure, Adipinsäure, Natriumstearylfumarat (Pruv^{®}) und/oder Magnesiumstearat dar.

Schmiermittel werden üblicherweise in einer Menge von 0,1 bis 3 Gew.-%, bezogen auf das Gesamtgewicht der Formulierung, verwendet.

In einer bevorzugten Ausführungsform umfasst folglich die erfindungsgemäße pharmazeutische Formulierung
(i) 50 bis 95 Gew.-% erfindungsgemäßes Intermediat, bevorzugt 60 bis 90 Gew.-% Intermediat,
(ii) 5 bis 25 Gew.-%, bevorzugt 8 bis 15 Gew.-% Sprengmittel,
(iii) 0 bis 50 Gew.-%, bevorzugt 10 bis 35 Gew.-% Füllstoff,

### bezogen auf das Gesamtgewicht der Formulierung.

Zudem ist es bevorzugt, dass die erfindungsgemäße pharmazeutische Formulierung noch einen oder mehrere weitere Wirkstoffe enthalten kann. In einer bevorzugten Ausführungsform enthält die erfindungsgemäße pharmazeutische Formulierung 0,1 bis 10 Gew.-%, mehr bevorzugt 0,5 bis 8 Gew.-%, eines weiteren Wirkstoffs, bezogen auf das Gesamtgewicht der Formulierung. Bei dem weiteren Wirkstoff handelt es sich z.B. um einen AT1-Rezeptorantagonisten, ACE-Inhibitor, Betablocker, Calciumkanalblocker, Aldosteronsynthaseinhibitor, Aldosteronrezeptorantagonisten oder bevorzugt um ein Diuretikum. Besonders bevorzugt handelt es sich um Hydrochlorthiazid (kurz HCT, HCTZ oder HCZ).

Die erfindungsgemäße pharmazeutische Formulierung wird bevorzugt zu Tabletten verpresst. Die erfindungsgemäßen Intermediate werden daher mittels Direktverpressung zu Tabletten verpresst oder vor dem Verpressen zur Tablette einer Granulierung, z.B. einer Trockengranulierung oder einer Feuchtgranulierung, unterworfen. Intermediate mit einer Schüttdichte von kleiner 0,5 g/ml werden bevorzugt per Trockengranulation oder Feuchtgranulation verarbeitet.

Die Tablettierbedingungen werden bevorzugt so gewählt, dass die resultierenden Tabletten ein Verhältnis von Tablettenhöhe zu Gewicht von 0,005 bis 0,3 mm/mg, besonders bevorzugt 0,05 bis 0,2 mm/mg, aufweisen.

Ferner weisen die resultierenden Tabletten bevorzugt eine Härte von 50 bis 200 N, besonders bevorzugt von 80 bis 150 N, auf. Die Härte wird gemäß Ph.Eur. 6.0, Abschnitt 2.9.8 bestimmt.

Zudem zeigen die resultierenden Tabletten bevorzugt eine Friabilität von kleiner 5 %, besonders bevorzugt von kleiner 2 %, insbesondere kleiner 1%, auf. Die Friabilität wird gemäß Ph.Eur. 6.0, Abschnitt 2.9.7 bestimmt.

Schließlich weisen die erfindungsgemäßen Tabletten üblicherweise eine "Content Uniformity" von 90 bis 110%, bevorzugt von 95 bis 105%, mehr bevorzugt von 98 bis 102%, insbesondere von 99 bis 101% vom durchschnittlichen Gehalt auf. Die "Content Uniformity" wird gemäß Ph.Eur.6.0, Abschnitt 2.9.6. bestimmt.

Das Freisetzungsprofil der erfindungsgemäßen Tabletten weist im Falle einer IR-Formulierung gemäß USP-Methode (paddle, 0,1 n HCl, 75 rpm) nach 10 Minuten üblicherweise einen freigesetzten Gehalt von mindestens 30%, bevorzugt mindestens 60%, insbesondere mindestens 80%, auf.

Das Freisetzungsprofil der erfindungsgemäßen Tabletten weist im Falle einer MR-Formulierung gemäß USP-Methode (paddle, 0,1 n HCl, 75 rpm) nach 60 Minuten üblicherweise einen freigesetzten Gehalt von nicht mehr als 20%, bevorzugt nicht mehr als 30%, insbesondere nicht mehr als 40%, auf. Innerhalb von 8 Stunden sind jedoch bevorzugt mindestens 80%, mehr bevorzugt mindestens 90%, freigesetzt.

Die vorstehenden Angaben zu Härte, Friabilität, Content Uniformity und Freisetzungsprofil beziehen sich hierbei bevorzugt auf die unbefilmte Tablette für eine IR-Formulierung. Für eine modified release Tablette bezieht sich das Freisetzungsprofil auf die Gesamtformulierung.

Bei den durch das erfindungsgemäße Verfahren hergestellten Tabletten kann es sich um Tabletten, die unzerkaut geschluckt werden (unbefilmt oder bevorzugt befilmt), handeln. Ebenfalls kann es sich um Kautabletten oder um Disperstabletten handeln. Unter "Disperstablette" wird hierbei eine Tablette zur Herstellung einer wässrigen Suspension zum Einnehmen verstanden.

Im Falle von Tabletten, die unzerkaut geschluckt werden, ist es bevorzugt, dass diese mit einer Filmschicht überzogen werden. Hierbei können die im Stand der Technik üblichen Verfahren zur Befilmung von Tabletten Anwendung finden. Die vorstehend genannten Verhältnisse von Wirkstoff zu Hilfsstoff beziehen sich jedoch auf die unlackierte Tablette.

Für die Befilmung werden bevorzugt makromolekulare Stoffe verwendet, beispielsweise modifizierte Cellulosen, Polymethacrylate, Polyvinylpyrrolidon, Polyvinylacetatphthalat, Zein und/oder Schellack oder natürliche Gummi, wie z.B. Carrageenan.

Die Schichtdicke des Überzugs beträgt bevorzugt 1 bis 100 *µ*m, mehr bevorzugt 5 bis 60 *µ*m.

Mögliche Darreichungsformen für die erfindungsgemäßen pharmazeutischen Formulierungen zur oralen Verabreichung beinhalten Tabletten, Kapseln, Dispersionen, Suspensionen und ähnliche Formulierungen. Tabletten sind bevorzugt. Die Dosierung des Wirkstoffs oder der Wirkstoffkombination kann beispielsweise aufgrund der Art der zu behandelnden Erkrankung, der Schwere der Erkrankung sowie der Darreichungsform variiert werden.

Zur Behandlung der Krankheiten, die für den Wirkstoff oder die Wirkstoffkombination in den erfindungsgemäßen Formulierungen angezeigt sind, werden gewöhnlich befriedigende Ergebnisse erzielt, wenn der enthaltende Wirkstoff Aliskiren in einer täglichen Dosierung von 10 mg bis 1000 mg verabreicht wird, bevorzugt von 50 mg bis 500 mg, besonders bevorzugt von 75 mg bis 300 mg und/oder wenn der optional enthaltene Wirkstoff HCT in einer täglichen Dosierung von 5 mg bis 50 mg, bevorzugt von 10 mg bis 25 mg, verabreicht wird. Weiterhin bevorzugt ist die einmal tägliche Verabreichung der täglichen Dosis oder die Verabreichung der täglichen Dosis, verteilt auf zwei bis fünf Dosierungen. In den gleichen Dosierungen sind auch Applikationen seltener als täglich, beispielsweise alle zwei, drei oder vier Tage, möglich, beispielsweise in einer Formulierung mit verzögerter Freisetzung. Das Dosierschema kann innerhalb oder auch außerhalb dieses Rahmens variiert werden, um optimale Behandlungsergebnisse zu erreichen.

Die erfindungsgemäßen pharmazeutischen Formulierungen zeichnen sich üblicherweise durch eine Freisetzung und Absorption aus, die zu vorteilhaften Werten der AUC (*"Area under curve",* Fläche unter der Plasmaspiegelkurve von 0 bis 48 Stunden nach peroraler Gabe), vorteilhaften Werten der Cmax (maximaler Plasmaspiegel) und vorteilhaften Werten der Tmax (Zeitpunkt des Erreichens des maximalen Plasmaspiegels nach peroraler Gabe) führen.

In einer bevorzugten Ausführungsform führt die perorale Verabreichung der erfindungsgemäßen Formulierungen an einen Menschen als Patienten zu einem Plasmaspiegel-Profil, das sich durch ein Tmax bezüglich des Wirkstoffs Aliskiren von etwa 0,5 bis 3 Stunden auszeichnet.

In einer bevorzugten Ausführungsform führt die perorale Verabreichung der erfindungsgemäßen Formulierungen an einen Menschen als Patienten zu einem Plasmaspiegel-Profil, das sich durch ein Tmax bezüglich des Wirkstoffs HCT von etwa 0,5 bis 3 Stunden auszeichnet.

In einer bevorzugten Ausführungsform führt die perorale Verabreichung der erfindungsgemäßen Formulierungen an einen Menschen als Patienten zu einem Plasmaspiegel-Profil, das sich durch ein Cmax bezüglich des Wirkstoffs Aliskiren von etwa 10 bis 90 ng/ml, bevorzugt 20 bis 80 ng/ml, auszeichnet.

In einer bevorzugten Ausführungsform führt die perorale Verabreichung der erfindungsgemäßen Formulierungen an einen Menschen als Patienten zu einem

Plasmaspiegel-Profil, das sich durch ein Cmax bezüglich des Wirkstoffs HCT von etwa 100 bis 200 ng/ml, bevorzugt 115 bis 185 ng/ml, auszeichnet.

In einer bevorzugten Ausführungsform führt die perorale Verabreichung der erfindungsgemäßen Formulierungen an einen Menschen als Patienten zu einem Plasmaspiegel-Profil, das sich durch eine AUC bezüglich des Wirkstoffs Aliskiren von etwa 300 bis 1500 ng-h/ml. bevorzugt 400 bis 1300 ng.h /ml, auszeichnet.

In einer bevorzugten Ausführungsform führt die perorale Verabreichung der erfindungsgemäßen Formulierungen an einen Menschen als Patienten zu einem Plasmaspiegel-Profil, das sich durch eine AUC bezüglich des Wirkstoffs HCT von etwa 500 bis 1500 ng.h /ml, bevorzugt 650 bis 1350 ng-h/ml, auszeichnet.

Die vorstehend genannten Plasmaspiegelwerte sind bevorzugt Mittelwerte, erhältlich durch Untersuchung von Blutproben einer Gruppe von 10 Probanden (mit durchschnittlich 70 kg Körpergewicht), wobei die entsprechenden Blutproben 0, 1, 3, 4, 6, 8, 24 und 48 Stunden nach peroraler Gabe der erfindungsgemäßen Formulierung entnommen wurden. Die Bestimmung der Plasmaspiegelwerte kann bevorzugt durch geeignete HPLC-MSMS-Methoden geschehen. Die Berechnung der AUC erfolgt beispielsweise durch ein Computerprogramm, wie das Programm Excel der Firma Microsoft.

In einer bevorzugten Ausführungsform werden die erfindungsgemäßen pharmazeutischen Formulierungen zur Behandlung der essentiellen Hypertonie oder der Herzinsuffizienz entweder als Monopräparat oder in Kombination mit Diuretika oder ACE-Hemmern eingesetzt. Besonders bevorzugt ist die Behandlung von Patientengruppen, bei denen die Therapie mit anderen blutdrucksenkenden Mitteln, ganz besonders bevorzugt die Monotherapie mit Diuretika oder ACE-Hemmern oder die Kombinationstherapie von Diuretika und ACE-Hemmern, nicht zu befriedigenden Ergebnissen geführt hat.

Gegenstand der Erfindung ist somit auch eine Tablette enthaltend 50 bis 500 mg Aliskirenbase und gegebenenfalls 5 bis 50 mg HCT, wobei die Tablette eine Härte von 50 bis 250 N, eine Friabilität von kleiner 5 % und eine Gleichförmigkeit des Gehalts von 95 bis 105 % aufweist, und wobei die Verabreichung bezüglich des Wirkstoffs Aliskiren zu einer Tmax von 0.5 bis 3 Stunden, zu einer Cmax von 10 bis 90 ng/ml sowie zu einer AUC von etwa 300 bis 1500 ng.h /ml führt, und wobei gegebenenfalls die Verabreichung bezüglich des Wirkstoffs HCT zu einer Tmax von 0,5 bis 3 Stunden, zu einer Cmax von 100 bis 200 ng/ml sowie zu einer AUC von etwa 500 bis 1500 ng.h/ml führt. In der erfindungsgemäßen Tablette liegt Aliskiren bevorzugt in Form des erfindungsgemäßen Intermediats vor. Entsprechend enthält die erfindungsgemäße Tablette bevorzugt eine erfindungsgemäße pharmazeutische Zusammensetzung, insbesondere gemäß einem der Ansprüche 10 bis 14. Die Verabreichung der erfindungsgemäßen Tablette erfolgt bevorzugt einmal täglich.

Die Erfindung soll anhand der nachfolgenden Beispiele veranschaulicht werden.

### BEISPIELE

### Beispiel 1

| | | |
|---|---|---|
| 1. | Aliskiren Base | 150 mg |
| 2. | Mikrokristalline Cellulose | 150 mg |
| 3. | EtOH 96% | q.s. |
| 4. | Mikrokristalline Cellulose | 100 mg |
| 5. | Aerosil^{®} 200 | 5 mg |
| 6. | Crospovidon | 30 mg |
| 7. | Magnesiumstearat | 15 mg |

Aliskirenbase wurde mit einer ausreichenden Menge Ethanol 96% gelöst und auf Pos. 2 aufgezogen. Nach einer ausreichenden Trocknung wurde die Mischung über ein 0,8 mm Sieb gegeben und die gesiebten (0,8 mm) Pos. 4 - 6 zugegeben. Die Mischung wurde 15 min im Turbula^{®} gemischt. Anschließend wurde Pos. 7 über ein Sieb (0,8 mm) zugeben und 5 min im Turbula^{®} gemischt.

Die fertige Mischung wurde zu Tabletten Ø 11 mm, Härte 90-120 N verpresst.

### Beispiel 2: Formulierungsbeispiel Aliskiren freie Base in Kombination mit HCT

| | | |
|---|---|---|
| 1. | Aliskiren Base | 300 mg |
| 2. | Di Cafos^{®} AN | 300 mg |
| 3. | Hydrochlorthiazid (HCT) | 12,5 mg |
| 4. | Di Cafos^{®} AN | 20 mg |
| 5. | Di Cafos^{®} AN | 50 mg |
| 6. | Aerosil^{®} 200 | 8 mg |
| 7. | Crospovidon | 40 mg |
| 8. | Magnesiumstearat | 10 mg |
| 9. | EtOH 96% | q.s. |

Aliskirenbase wurde in EtOH gelöst und Di Cafos^{®} AN (Pos. 2) mit dieser Lösung gemischt und getrocknet. Nach der Trocknung wurde die Mischung über ein Sieb (0,8 mm) gesiebt und mit Di Cafos^{®} AN (Pos. 5) 10 min im Turbula^{®} gemischt. HCT und Di Cafos^{®} AN (Pos. 4) wurden über ein Sieb (0,8 mm) gegeben und 10 min im Turbula^{®} gemischt. Die Mischung wurde zu der getrockneten und gesiebten Mischung aus Pos.1, 2 und 5 gegeben, Aerosil^{®} 200 und Crospovidon wurden über Sieb zugegeben und 10 min im Turbula^{®} gemischt. Magnesiumstearat wurde über Sieb zugegeben zugeben und 5 min gemischt.

Die Mischung wurde mit der Rundläuferpresse (Riva) zu Tabletten oblong 18 x 8,5 mm mit 50-120 N Härte verpresst.

### Beispiel 3: in-vitro Freisetzung

Die in-vitro Freisetzung der Tabletten gemäß Beispiel 1 wurde untersucht (USP Methode II, paddle, 500 ml 0,1 N HCl, pH 1,1, 37 °C, 75 UpM).

Die erfindungsgemäßen Tabletten zeigten nach 5 Minuten 99 % Freisetzung und nach 10 Minuten 100 % Freisetzung.

## Patentansprüche

1. Intermediat, enthaltend Aliskirenbase und Trägerstoff, wobei Aliskirenbase als feste Dispersion vorliegt.

2. Intermediat gemäß Anspruch 1, wobei Aliskirenbase auf den Trägerstoff aufgebracht und/oder in den Trägerstoff eingelagert ist.

3. Intermediat gemäß Anspruch 1 oder 2, wobei das Gewichtsverhältnis von Aliskirenbase zu Trägerstoff 10 : 1 bis 1 : 10 beträgt.

4. Intermediat gemäß einem der Anspruch 1 bis 3, wobei es sich bei dem Trägerstoff um einen spröden Trägerstoff mit einem Yield Pressure von mehr als 80 MPa handelt.

5. Intermediat nach einem der Ansprüche 1 bis 4, wobei ein nicht-wasserlöslicher Trägerstoff verwendet wird.

6. Intermediat nach einem der Ansprüche 1 bis 5, wobei ein quellbarer Trägerstoff verwendet wird.

7. Verfahren zur Herstellung eines Intermediats, enthaltend Aliskiren und Trägerstoff, wobei Aliskirenbase als feste Dispersion vorliegt, umfassend die Schritte
(a) Lösen von Aliskirenbase in einem Lösungsmittel oder Lösungsmittelgemisch und Inkontaktbringen der Lösung mit einem Trägerstoff,
(b) Entfernen des Lösungsmittels oder Lösungsmittelgemisches,
(c) gegebenenfalls Granulation des in Schritt (b) erhaltenen Gemisches.

8. Verfahren gemäß Anspruch 7, wobei Schritt (b) als Gefriertrocknungsschritt oder Sprühtrocknungsschritt erfolgt.

9. Verfahren gemäß Anspruch 7, wobei die Schritte (b) und (c) im Wirbelschichtgranulator erfolgen.

10. Intermediat, erhältlich nach einem Verfahren gemäß einem der Ansprüche 7 bis 9.

11. Pharmazeutische Formulierung, enthaltend Aliskirenbase in Form eines Intermediats gemäß einem der Ansprüche 1 bis 6 und 10, sowie gegebenenfalls mindestens einen weiteren pharmazeutischen Hilfsstoff.

12. Pharmazeutische Formulierung gemäß Anspruch 11 zur oralen Verabreichung, bevorzugt in Form einer Tablette.

13. Pharmazeutische Formulierung gemäß Anspruch 11 oder 12, enthaltend
(i) 50 bis 95 Gew.-% Intermediat,
(ii) 5 bis 25 Gew.-% Sprengmittel,
(iii) 0 bis 50 Gew.-% Füllstoff,
bezogen auf das Gesamtgewicht der Formulierung.

14. Pharmazeutische Formulierung gemäß einem der Ansprüche 11 bis 13, enthaltend 0,1 bis 10 Gew.-% eines weiteren Wirkstoffs, bevorzugt Hydrochlorthiazid.

15. Tablette enthaltend 50 bis 500 mg Aliskirenbase, wobei die Tablette eine Härte von 50 bis 250 N, eine Friabilität von kleiner 5% und eine Gleichförmigkeit des Gehalts von 95 bis 105% aufweist,
wobei die Verabreichung bezüglich des Wirkstoffs Aliskiren zu einer Tmax von 0.5 bis 3 Stunden, zu einer Cmax von 10 bis 90 ng/ml sowie zu einer AUC von etwa 300 bis 1500 ng.h /ml führt, und wobei die Verabreichung ein- bis zweimal täglich erfolgt.
